Europäisches Patentamt

European Patent Office

Office européen des brevets

(11) Publication number: **0 216 747**
A2

# (12) EUROPEAN PATENT APPLICATION

(21) Application number: 86870137.6

(22) Date of filing: 26.09.86

(51) Int. Cl.⁴: **C 12 N 15/00**
C 12 P 21/02, C 12 N 1/20
//(C12N1/20,C12R1:19)

(30) Priority: 27.09.85 US 781290

(43) Date of publication of application:
01.04.87 Bulletin 87/14

(84) Designated Contracting States:
AT BE CH DE FR GB IT LI LU NL SE

(71) Applicant: **SMITHKLINE BECKMAN CORPORATION**
**One Franklin plaza**
**Philadelphia Pennsylvania 19103 (US)**

(72) Inventor: **Debouck, Christine Marie**
**667 Pugh Road**
**Wayne Pennsylvania 19087 (US)**

**Ho, Yen Sen**
**362 Old State Road**
**Verwin Pennsylvania 19312 (US)**

**Rosenberg, Martin**
**1280 Wisteria Drive**
**Malvern Pennsylvania 19355 (US)**

(74) Representative: **Tasset, Gérard**
**SMITHKLINE - RIT rue de l'Institut, 89**
**B-1330 Rixensart (BE)**

(54) Proteolytic deficient E. coli.

(57) This invention relates to a method of stably expressing a foreign polypeptide coding sequence which is not stably expressed by a htpR+ host due to degradation by proteases under the control of the htpR+ gene which comprises transforming a htpR- mutant host with a plasmid containing the foreign polypeptide coding sequence operatively linked to expression control sequences, and culturing the transformed host in an appropriate medium; or transforming a htpR+ host with the coding sequence and then changing the host phenotype to a htpR- mutant prior to culturing in an appropriate medium; and to the hosts transformed according to such methods.

EP 0 216 747 A2

Description

# PROTEOLYTIC DEFICIENT E. coli

BACKGROUND OF THE INVENTION

This invention relates to a method of stably expressing a foreign polypeptide coding sequence which is not stably expressed by a htpR+ host due to degradation by proteases under the control of the htpR+ gene product.

The htpR (also known as rpoH or hin) gene encodes a 32-kilodalton sigma factor ($\sigma^{32}$) that promotes transcription from heat-shock promoters. When htpR+ E. coli cells are transferred to high temperature, the rate of synthesis of a small number of proteins increases. This physiological response to temperature shift is known as the heat-shock response. Since many other stimuli including ultraviolet light, amino acid starvation, nalidixic acid, coumermycin and ethanol induce the synthesis of heat shock proteins (hsp's) in E. coli, the response may be part of a general cellular mechanism for adaptation to stress. At least one protease, lon, has been demonstrated to be a hsp. [see Goff et al., Cell, 41, 587-595 (1985); Goff et al., Proc. Natl. Acad. Sci., U.S.A., 81, 6647-6651 (1984); and Phillips et al., J. Bacteriology, 159(1), 283-287 (1984)].

The heat-shock response in E. coli was initially defined by an analysis of the changes in rates of synthesis of individual proteins after shift to high temperature. To date, 17 proteins produced by E. coli have been characterized as heat-shock proteins. Immediately after shift to high temperature, the rates of synthesis by E. coli of individual hsp's increase approximately 5 to 20 fold, depending on the protein. The increased rate of synthesis peaks at 5-10 minutes and then declines by 30 minutes after temperature upshift to a new steady state rate of synthesis somewhat greater than that at low temperature. Where it has been examined, the increased synthesis of heat shock proteins has been shown to be accompanied by an increase in the rate of synthesis of their mRNAs. Thus, initiation of the heat-shock response is regulated, at least in part, at the transcriptional level.

Neidhardt et al., J. Bacteriol., 153, 597-603 (1983) (Neidhardt I), Yamamori et al., Proc. Nat'l Acad. Sci. USA, 79, 860-864 (1982), Neidhardt et al., Biochem. Biophys. Res. Commun., 100, 894-900 (1981) (Neidhardt II), and Bachmann, Microbiol. Rev., 47, 180-230 (1983), disclose that the htpR gene, mapping at approximately 76 min on the E. coli chromosome, is involved in regulation of the heat-shock response. Yamamori et al., Neidhardt II, and Cooper et al., Mol. Gen. Genet., 139 167-176 (1975), disclose that this gene is defined by an amber mutation htpR165 (hin165) carried in a strain which contains a temperature sensitive (ts) suppressor tRNA. Cells containing this mutation are ts for growth, and fail to induce synthesis of hsp's upon temperature upshift. Using a series of suppressors of varying efficiency, all of which insert the same amino acid, Yamamori et al. have shown that the efficiency of suppression of the htpR165 mutation is correlated with both the rate of synthesis of hsp's at the peak of the response and the permissive growth temperature. Yamamori et al. and Cooper et al. have shown that an efficient suppressor functional at all temperatures restores both the heat-shock response and high temperature growth. Thus, inefficient suppression of the htpR165 mutation blocks the heat-shock response. Cell death ensues at high temperature.

The 70-kilodalton sigma subunit ($\sigma^{70}$) of E. coli RNA polymerase holoenzyme plays an essential role in regulating transcription initiation and maintenance of cell growth. Several mutations in rpoD, the E. coli gene that encodes $\sigma^{70}$, including rpoD800 (rpoD285), lead to a temperature-sensitive growth phenotype (ts). Grossman et al., Cell, 32, 151-159 (1983), disclose that the mutant sigma subunit of RNA polymerase encoded by the rpoD800 allele of the E. coli rpoD gene is rapidly degraded at high temperature. Baker et al., Proc. Natl. Acad. Sci. USA, 81, 6779-6783 (1984) disclose that whereas the mutant sigma protein is rapidly degraded in htpR+ strains at 42°C (T 1/2 = 6 minutes at 42°C), it is quite stable in isogenic htpR- mutant strains at 42°C (T 1/2 = 60 minutes at 42°C). Baker et al. also report that the rate of degrada-tion of mutant sigma in htpR- mutant strains is indistinguishable from that of the wild type sigma in htpR- strains. Furthermore, Baker et al. also report that the rate of degradation of the mutant sigma protein at 30°C was significantly less in htpR- mutant strains (T 1/2 = 60 minutes) than in isogenic htpR+ strains (T 1/2 - 20 minutes).

Welply et al., Biochem. Biophys. Res. Commun., 93, 223-227 (1980), disclose that the lacZX90 mutation is an ochre nonsense mutation located near the 3' end of the E. colilacZ gene. Baker et al., cited above, disclose that the almost full length β-galactosidase polypeptide encoded by the lacZX90 gene is unstable in htpR+ strains at both 30° and 42°C (T 1/2 = 7 minutes at 42°C), but is quite stable in htpR- mutant strains at both 30° and 42°C (T 1/2 = 60 minutes at 42°C). Baker et al. also disclose that the rate of degradation of lacZX90 gene product in htpR- mutant strains is the same as the rate of degradation of wild type lacZ gene product in htpR- mutant and htpR+ strains.

Grossman et al., J. Bacteriol., 161(3), 939-943 (1985), disclose that the same defect in the proteolysis of the rpoD800 (rpoD825) gene product is exhibited by htpR- (rpoH-) cells which have both a htpR107 mutation, a htpR111mutation, a htpR112 mutation or a htpR113 mutation and the rpoD800 gene. The htpR112 mutation is defined by a missense mutation causing a leucine-to-tryptophan change seven amino acids from the carboxyl terminus of the htpR (rpoH) gene product. Cells with the htpR112 mutation and the rpoD800 gene have also been found to be defective in the proteolysis of the lacZX90 gene product (See, Grossman et al., cited above).

The product of the lon gene E. coli, La, a hsp, plays an important role in the degradation of abnormal proteins. To determine whether the presence of abnormal proteins stimulates expression of this gene, Goff et al., Cell, 41, 587-595 (1985), examined its transcrip tion using a lon-lacZ operon fusion and disclose that after the cells synthesized large amounts of aberrant polypeptides (e.g. following incorporation of the arginine analog, canavanine, or production of incomplete proteins with puromycin, or induction of translational errors with streptomycin), these cells showed a two to three fold increase in lon-lacZ expression. Goff et al. also disclose that synthesis of a single cloned protein, e.g. human tissue plasminogen activator, caused a similar increase in lon transcription and that this induction of lon by abnormal proteins requires the heat-shock regulatory gene htpR and was not seen in htpR- cells. Goff et al. also disclose that under these various conditions, other heat-shock proteins were also induced, and conclude that the appearance of aberrant cell proteins may be a common signal under many adverse conditions for the induction of cell protease (or proteases) and other heat-shock proteins.

It has now been unpredictably discovered that the findings of Grossman et al., cited above, and Baker et al., cited above (i.e., that htpR- mutants can stabilize the expression of those E. coli derived polypeptide sequences which are not stably expressed in htpR+ hosts due to degradation by htpR+ controlled proteases) are extendable to foreign polypeptide sequences, i.e., htpR- mutants can stabilize the expression of those non-E. coli derived (foreign) polypeptide sequences which are not stably expressed in htpR+ hosts due to degradation by htpR+ controlled proteases. Such foreign coding sequences can be derived from prokaryotic organisms other than E. coli, eukaryotic organisms or cells, or viruses.

## SUMMARY OF THE INVENTION

This invention relates to a method of stably expressing a foreign polypeptide coding sequence not stably expressed in htpR+ E. coli strains due to degradation by proteases under the control of the htpR+ gene which comprises (a) transforming a host htpR- mutant cell with a DNA molecule containing the polypeptide coding sequence operatively linked to an expression control sequence, and (b) culturing the transformed host in an appropriate medium under conditions such that the coding sequence is expressed.

This invention also relates to a method of stably expressing a foreign polypeptide coding sequence not stably expressed in htpR+ strains due to degradation by proteases under the control of htpR+ gene which comprises (a) transforming a host htpR+ cell with a DNA molecule containing the polypeptide coding sequence operatively linked to an expression control sequence, (b) changing the phenotype of the htpR+ cell to a htpR- mutant cell, and (c) culturing the htpR+ mutant cell in an appropriate medium under conditions such that the coding sequence is expressed.

This invention also relates to the htpR- mutant hosts transformed by either of such methods.

## DETAILED DESCRIPTION OF THE INVENTION

This invention generally relates to a method of stably expressing a foreign polypeptide coding sequence which is not stably expressed in a htpR+ strain due to degradation by proteases under the control of the htpR+ gene.

By the term "htpR- mutant" is meant an E. coli cell with a mutation in the htpR gene. Such htpR- mutant may be one which carries no suppressor of the htpR- mutation, such as the htpR- mutant strains described in Yura et al., Proc. Natl. Acad. Sci. USA, 81, 6803-6807 (1984). Such htpR- mutant is preferably a conditional mutant. For example, if an E. coli cell known as a htpR165 mutant also carries a temperature-sensitive suppressor known as supCts, it is a htpR- conditional mutant. The supCts encoded suppressor is an inefficient suppressor of the htpR165 mutation, and operates to keep the htpR165 cell viable at high temperature. See, Baker et al., Proc. Natl. Acad. Sci. U.S.A., 81, 6779-6783 (1984). Such htpR- mutant is also preferably one which carries a gene capable of pseudocomplementation of the htpR- mutation. For example, a htpR- E. coli cell known as a htpR112 mutant also carries the rpoD800 gene. The rpoD800 gene product operates to keep the htpR112 cell viable at low or high temperature. Similarly, the E. coli htpR- mutants known as htpR107, htpR111 or htpR113 also carry the rpoD800 gene. See, Grossman et al., J. Bacteriol., 161 (3), 939-943 (1985).

By the term "foreign polypeptide coding sequence" is meant a polypeptide coding sequence derived from any source other than E. coli, including other prokaryotes, eukaryotes or viruses. Preferred polypeptide coding sequences are from eukaryotic sources, particularly mammalian, and viral coding sequences which express in mammalian cells. Preferred polypeptide coding sequences are those which code for polypeptides of pharmaceutical importance which are susceptible to degradation by htpR+ strains. By the term "expression control sequence" is meant a sequence necessary for expression of the foreign polypeptide coding sequence such as a promoter or other regulatory region. By the term "promoter" is meant any region upstream of the foreign polypeptide coding sequence which permits binding of RNA polymerase and transcription to occur. By the term "regulatory region" is meant any region which regulates the transcription or the translation of the foreign polypeptide coding sequence. By the term "appropriate medium" is meant a culture medium which will enable the expression of the foreign polypeptide coding sequence by a host htpR- cell transformed therewith. By the term "stably expressed" is meant that the protein of interest accumulates to $> .1\%$ of total cellular protein, or in an amount which is at least about 10-fold greater than the amount in which the polypeptide accumulates in a htpR+ E. coli host. Such protein accumulation can be determined by conventional techniques

3

such as the $^{35}$S-L-methionine pulse-chase procedure of Rosenberg et al., Methods Enzymol., 101, 123-138 (1983). followed by SDS-Page (polyacrylamide gel electrophoresis) and autoradiography, or SDS-Page and Coomassie blue staining. Levels of accumulation are determined by densitometer tracing of the gels.

It is well known that a mutation in a gene, other than a deletion mutation, may lead to leakiness, i.e., low level expression of the gene's product. Thus, one of skill in the art would expect that a htpR− mutant, other than a htpR− deletion mutant, may still produce a low level of the htpR gene product which may lead to low level activation of lon and/or other heat-shock genes controlled by the htpR gene product. Furthermore, even in the total absence of the htpR gene product, one of skill in the art would expect that the promoter of lon and/or other heat-shock genes controlled by the htpR gene product may still lead to some low level production of the heat-shock gene product due to the basal level of transcription determined by the promoter. However, such low level of lon and/or other heat-shock gene protein which may be produced by a htpR− mutant does not prevent the stabilization of expression in host htpR− cells of foreign polypeptide coding sequences which would not be stably expressed in htpR+ hosts due to degradation by htpR controlled proteases. Thus, the method of this invention is useful in eliminating or substantially eliminating the effect of those proteases which cause rapid degradation of foreign gene products expressed by htpR+ E. coli transformed with such genes, and for overproducing those recombinant gene products which are not stably expressed by htpR+ hosts due to degradation by htpR+ controlled proteases.

Any DNA molecule which (a) does not contain a coding sequence which will effectively suppress or complement the htpR− mutation, (b) contains a foreign polypeptide coding sequence operatively linked to an expression control sequence, and (c) can be stably maintained, extrachromosomally, by a htpR− mutant host transformed therewith, can be employed in the method of the subject invention. By the term "effectively suppress or complement the htpR− mutation" is meant restoring of the htpR+ phenotype, i.e. normal heat-shock response and proteolysis of proteins stabilized in htpR− cells. For example, if the host htpR− cell is a htpR165 mutant, the DNA fragment must not contain the supF (SulII) suppressor or an intact htpR gene [See, Baker et al., Proc. Natl. Acad. Sci. USA, 81, 6779-6783 (1984)]; or if the host htpR− cell is a htpR107, htpR111, htpR112 or htpR113 mutant, the plasmid must not contain an intact htpR gene [See, Grossman et al., J. Bacteriol., 161(3), 939-943 (1985)]. Preferred DNA molecules are those derived from P$_L$ vectors known conventionally to be useful as E. coli expression vectors, such as pAS1 or pKC30. pKC30 is described by Shimatake et al., Nature, 292(5819), 128-132 (1981). pAS1 is described in Rosenberg et al., Methods Enzymol., 101, 123138 (1983). The desired foreign polypeptide coding sequence can be obtained and isolated from its source by conventional techniques. The foreign polypeptide coding sequence can be ligated to the DNA fragment and operatively linked to an expression con- trol sequence by conventional techniques. The htpR− conditional mutant host can be transformed with the DNA molecule by conventional techniques. Alternatively, a htpR+ host can be transformed with the DNA molecule by conventional techniques, and then changed to the hptR− conditional mutant phenotype by conventional techniques such as by isolating the htpR− mutation directly by the method of Cooper et al., Mol. Gen. Genet., 139, 167-176 (1975), or by localized mutagenesis, such as the method described by Tobe et al., Mol. Gen. Genet., 195, 10-16 (1984). Alternatively, a well defined htpR-mutation, such as htpR165, can be moved into a cell line of interest by convention techniques such as conjugation, or transduction with or without the help of a transposon [See, Miller, "Experiments in Molecular Genetics", Cold Spring Harbor Laboratory (1972); Silhavy et al., "Experiments with Gene Fusions", Cold Spring Harbor Laboratory (1984)]. Finally, the transformed htpR− mutant host is cultured in an appropriate medium, i.e., a nutrient broth which will enable expression of the foreign polypeptide coding sequence, by conventional techniques. The conditions of the culture are such that the coding sequence is expressed, i.e., nutrients, temperature, pH and aeration are adjusted to conditions in which transcription and translation of the coding sequence are favored. After culturing under such conditions, the polypeptide may be recovered by conventional protein isolation and recovery techniques.

The htpR gene (also known as rpoH or hin) encodes a 32-kilodalton sigma factor ($\sigma^{32}$) that promotes transcription from heat-shock promoters and is required for normal heat-shock response [See, Grossman et al., Cell, 38, 383-390 (1984)]. A htpR− gene, due to at least one mutation in the htpR coding sequence, does not code for wild-type $\sigma^{32}$. Thus, htpR− strains are unable to undergo normal heat-shock response. Such strains are also able to stabilize expression of foreign polypeptide coding sequences which are not stably expressed in htpR+ hosts. Any strain which is phenotypically a htpR− mutant and which does not contain a coding sequence which will effectively suppress or complement the htpR−mutation can be used in the method of the subject invention. Such htpR− mutant hosts are known. Preferred htpR− hosts include those containing both the htpR165 mutation and the supC$^{ts}$ suppressor [See, e.g., Baker et al., cited above] and those containing both the htpR107, htpR111, htpR112 or htpR113 mutation and the rpoD800 gene [See, Grossman et al. J. Bacteriol., 161(3), 939-943 (1985)].

The 70-kilodalton sigma subunit ($\sigma^{70}$) of E. coli RNA polymerase holoenzyme plays an essential role in regulating transcription initiation and maintenance of cell growth. Several mutations in rpoD, the E. coli gene that encodes $\sigma^{70}$, including rpoD800 (rpoD285), lead to a temperature-sensitive growth phenotype (ts).

As reported by Grossman et al., Cell, 32, 151-159 (1983), the mutant sigma subunit of RNA polymerase encoded by the rpoD800 allele of the E. coli rpoD gene is rapidly degraded at high temperature. Baker et al., Proc. Natl. Acad. Sci. USA, 81, 6779-6783 (1984) disclose that whereas the mutant sigma protein is rapidly degraded in htpR+ mutant strains at 42°C (T 1/2 = 6 minutes at 42°C), it is quite stable in htpR- mutant strains at 42°C (T 1/2 = 60 minutes at 42°C). Baker et al. also report that the rate of degradation of mutant sigma in htpR- mutant strains is indistinguishable from that of the wild type sigma in htpR- strains. Furthermore, Baker et al. also report that the rate of degradation of the mutant sigma protein at 30°C was significantly less in htpR- mutant strains (T 1/2 = 60 minutes) than in htpR- strains (T 1/2 = 20 minutes).

As reported by Welply et al., Biochem. Biophys. Res. Commun., 93, 223-227 (1980), the lacZX90 mutation is an ochre nonsense mutation located near the 3' end of the E. coli lacz gene. Baker et al., cited above, disclose that the almost full length β-galactosidase polypeptide encoded by the lacZX90 gene is unstable in htpR+ strains at both 30° and 42°C (T 1/2 = 7 minutes at 42°C), but is quite stable in htpR- mutant strains at both 30° and 42°C (T 1/2 = 60 minutes at 42°C). Baker et al. also disclose that the rate of degradation of lacZX90 gene product in htpR- mutant strains is the same as the rate of degradation of wild type lacZ gene product in htpR- mutant and htpR+ strains.

It is important to note that the proteolytic defect manifested by htpR- mutant cells is not limited to those strains with the htpR165 mutation and the supCts suppressor. As disclosed by Grossman et al., J. Bacteriol., 161(3), 939-943 (1985), the same defect in the proteolysis of the rpoD800 (rpoD285) gene product is exhibited by htpR- (rpoH-) cells which have both a htpR107 mutation, a htpR111 mutation, a htpR112 mutation or a htpR113 mutation and the rpoD800 gene. The htpR112 mutation is defined by a missense mutation causing a leucine-to-tryptophan change seven amino acids from the carboxyl terminus of the htpR (rpoH) gene product. Cells with the htpR112 mutation and the rpoD800 gene have also been found to be defective in the proteolysis of the lacZX90 gene product (See, Grossman et al., cited above).

It has now been unpredictably discovered that the findings of Grossman et al., cited above, and Baker et al., cited above (i.e., that htpR- mutants can stabilize the expression of those E. coli derived polypeptide sequences which are not stably expressed in htpR+ hosts due to degradation by htpR+ controlled proteases) are extendable to foreign polypeptide sequences, i.e., htpR- mutants can stabilize the expression of those non-E. coli derived (foreign) polypeptide sequences which are not stably expressed in htpR+ hosts due to degradation by htpR+ controlled proteases.

cII protein of bacteriophage λ is a positive regulatory protein of transcription. The cII gene was cloned into a plasmid vector, pKC30, in such a way that its expression was under the control of a strong, regulatable λ phage promoter P_L [See, Shimatake et al., Nature, 292, (5819) 128-132 (1981)]. Such vector was transformed into λ lysogens to assess gene expression by heat induction. More than 30 of the point mutations of cII protein, which cover the different regions of the entire structural gene of cII protein, were also cloned into pKC30 and transformed into lysogens to assess gene expression by heat induction. The lysogens employed were either htpR+ or htpR- mutants. Surprisingly, most of these single amino acid alterations led to highly unstable polypeptides in htpR+ bacteria, i.e., the cII mutant polypeptides were much more unstable in htpR+ cells than was the wild type cII protein. The half-lives of most of the cII mutant proteins were 3-5 minutes versus 20 minutes for the wild type cII protein in htpR+ strains as determined by $^{35}$S-L-methionine pulse-chase assay [See, Rosenberg et al., Meth. in Enzymology, 101, 123-138 (1983)]. Several of the half-lives of the cII mutant proteins were even less than 1 minute in htpR+ strains. AR67, a lysogen constructed from the CAG456 cell line, carries the htpR165 mutation and the supCts suppressor, and was the only strain employed which was a htpR- mutant. AR67 exhibited a striking effect of stabilization upon these very unstable mutant cII proteins. For example, the half-life of a most unstable mutant cII protein, cII 3085, was extended from 0.5 min in htpR+ cell lines to at least 20 minutes in AR67 (htpR- mutant). In fact, the half-lives of all the mutant cII proteins assayed in AR67 were increased to at least the same level as the wild type cII protein. At the same time, AR65, a lysogen which is isogenic to AR67 but does not contain the htpR- mutation (i.e., it is htpR+), was unable to stabilize any of the mutant cII proteins. These results indicate that a htpR- mutant cell line (such as AR67) stabilizes a family of mutant cII proteins whose expression is not stable in htpR+ strains. The proteolytic deficiency of htpR- mutant strains shows certain selectivity since AR67 had no effect on the stability of expression of wild type cII protein as compared with its expression in htpR+ strains. However, although wild type cII protein is not really stably expressed in htpR+ strains, it is more stably expressed in htpR+ strains than are mutant cII proteins. Thus, selectivity may be due to the fact that the normal degradation of wild type cII protein is not under the control of the htpR gene, but is instead degraded by other proteolytic enzymes not related to the heat-shock response (i.e., not under regulation by the htpR gene).

Full length and several truncated versions of the yeast GAL4 coding sequence were cloned into a vector (pAS1) resulting in several clones encoding both the full length and truncated versions of the GAL4 protein. All versions were expressed in a htpR+ strain, AR66, and an isogenic htpR- mutant strain, AR68. The protein was not expressed stably by the AR66 host since it did not accumulate to > .1% of total cellular protein. Only the AR68 transformants showed accumulation of the protein products upon heat induction. Thus, the htpR mutation present in AR68 was essential for the accumulation, and thus stabilization, of the GAL4 gene product in E. coli.

The human c-myc coding region was taken from a full length cDNA clone and inserted into pOTS, a vector designed to express foreign gene products efficiently in E. coli. Three different htpR+ strains, N5151, AR66 and AR58, were transformed with the pOTS vector containing the human c-myc coding region. A htpR-

mutant strain, AR68, was also transformed with the pOTS-c-myc vector. AR66 is the htpR+ isogen of AR68. Although the htpR+ strains transformed with the pOTS-c-myc vector expressed the c-myc product. as indicated by SDS-gel electrophoresis of pulse labeled E. coli extracts, degradation of the protein was so rapid that it could not accumulate and be detected by Coomassie staining of the gel. Thus, the c-myc product was not expressed stably by the htpR+ host. On the other hand, the htpR- conditional mutant transformant accumulated the c-myc protein at a high level, and upon exposure to temperatures of 42°C for up to 120 minutes, Coomassie blue-staining of the gel showed that the induced product had accumulated to over 10% of the total htpR- conditional mutant protein content during the 2 hour time period. Thus, the method of this invention can be used to express protooncogene products which are not stably expressed by htpR+ cells. Such protooncogene products are useful for (i) preparation of monoclonal antibodies useful for detection of protooncogene products in cells, and (ii) as targets for pharmaceutical antagonists and/or agonists.

The monkey metallothionein gene and the mouse metallothionein gene were inserted into pAS1 by the method of Shatzman et al., Chapter Two, "Experimental Manipulation of Gene Expression", Academic Press, Inc. (1983), and Rosenberg et al., Methods in Enz., 101, 123-138 (1983). The resulting vectors were transformed into htpR+ strains [(AR58, N5151, AR13, C600 (λcl857)] and into htpR- mutant strains (AR67 and AR68) according to the method of Rosenberg et al., cited above. Pulse labeling of the gene products (performed according to the method of Rosenberg et al., cited above, except that cysteine was used instead of methionine) indicated that the mouse and monkey metallothionein proteins were expressed well in the htpR+ cell lines, but pulse chase data revealed that such proteins were very unstable in the htpR+ hosts and had a half-life of 2 minutes or less. The accumulation of these gene products was not enhanced by carrying out their synthesis in AR67 or AR68 (htpR- conditional mutant strains). In fact, the half-life of mouse and monkey metallothionein gene products expressed in htpR- strains was equally as short as in the htpR+ strains. These results indicate that the protease(s) responsible for the degradation of the mouse and monkey metallothionein gene products, are not under the control of the htpR gene.

EXAMPLES

In the following examples, specific embodiments of the invention are more fully disclosed. These examples are intended to be illustrative of the subject invention and should not be construed as limiting its scope. In all Examples, temperature is in degrees Centigrade (°C).

Further elaboration of the procedures of Examples 1 to 3 can be found in Baker et al., Proc. Natl. Acad. Sci. USA, 81, 6779-6783 (1984), the disclosure of which is hereby incorporated by reference.

Further elaboration of the procedures of Example 4 can be found in Watt et al., Molecular & Cell. Biol., 5(3), 448-456 (1985), the disclosure of which is hereby incorporated by reference.

The genotype and source of all bacterial strains referred to in the Examples is found in the following Table A:

## TABLE A

| Strain | Genotype |
|---|---|
| SC122 [a] | lac(am), trp(am), pho(am), supC[ts], rpsL, mal(am) |
| K165 [a] | SC122 htpR165 |
| CAG456 [b] | SC122 htpR165 |
| CAG510 [c] | SC122 rpoD800 - Tn10 |
| CAG481 [c] | CAG456 rpoD800 - Tn10 |
| CAG482 | CAG481 Ø80 |
| CAG483 | CAG481 Ø80 SuIII (SupF) |
| CAG603 [d] | SC122 proA/B::Tn10/F'proAB$^+$, lacZX90 |
| CAG604 [d] | CAG456 proA/B::Tn10/F'proAB$^+$, lacZX90 |
| CAG5107 | CAG604 pFN92 |
| AR65 [e] | SC122 ($\lambda$cI857kil$^-$$\Delta$HI)galE::Tn10 |
| AR67 [e] | CAG456 ($\lambda$cI857kil$^-$$\Delta$HI)galE::Tn10 |
| AR66 [g] | SC122 ($\lambda$cI857 Bam58-71$\Delta$HI)galE::Tn10 |
| AR68 [g] | CAG456 ($\lambda$cI857 Bam58-71$\Delta$HI)galE::Tn10 |
| AR58 | N99 [i] ($\lambda$cI857kil$^-$$\Delta$HI)galE::Tn10 |
| AR13 | C600 [j] ($\lambda$cI857kil$^-$$\Delta$HI)galE::Tn10 |
| N5151 | SA500 [k] his, ilv $\Delta$8 ($\lambda$cI857$\Delta$Bam 58-71$\Delta$HI) galk |
| UC5822 | N99 [i] ($\lambda$int 6 red 3cI857 cII3067 Pam3) |

(a)   Described in Cooper et al., Mol. Gen. Genet., 139, 167-176 (1975).

(b)   CAG456 was made by tranducing the htpR165 mutation linked to malT$^+$ into an SC122 derivative that was malT$^-$.

(c)   The Tn10 is $\sim$ 90% linked to rpoD [See, Grossman et al., Cell, 32, 151-159 (1983)].

(d)   The F' is from strain CSH21 [See, Miller et al., "Experiments in Molecular Genetics", Cold Spring Harbor Laboratory].

(e)   Derived by P1 galE::Tn10 cotransduction of the defective and temperature-sensitive prophage ($\lambda$ CI857kil$^-$$\Delta$HI) from AR14 [f], selecting for tetracycline resistance, and testing for $\lambda$ lysogeny, all according to the method of "Experiments with Gene Fusions", Silhavy et al., eds., Cold Spring Harbor Laboratory (1984).

## TABLE A (cont'd)

(f.)   AR14 is derived from C600 and contains a λ cI857kil⁻Δ HI defective prophage flanked by a galE::Tn10.

(g)   Derived by P₁ galE::Tn10 cotransduction of the defective and thermosensitive prophage (λcI857Δ BamΔ HI) from AR18 [h], selecting for tetracycline resistance and testing for λ lysogeny, all according to the method of "Experiments with Gene Fusions", Silhavy et al., eds.; Cold Spring Harbor Laboratory (1984). AR66 and AR68 differ from AR65 and AR67 by an additional prophage deletion (ΔBam58-71) which removes the cIII gene, among others.

(h)   AR18 is derived from N5151 and carries a galE::Tn10 adjacent to the λcI857Δ Bam Δ Hl prophage.

(i)   N99 is described in Lederberg, "Microbiological Genetics-10th Symposium Of The Society For General Microbiology", Hayes and Clowes eds., Cambridge University Press, 115-131 (1960).

(j)   C600 is described in Appleyard et al., Virology, 2, 565 (1956).

(k)   SA500 is described in Nakanishi et al., Cell, 3, 39-46 (1974).

EXAMPLE 1 htpR – CONDITIONAL MUTANT STRAINS ARE DEFECTIVE IN PROTEOLYSIS OF MUTANT E. COLI PROTEINS

A. Degradation of mutant sigma (encoded by the rpoD800 sigma allele).

The 70-kilodalton sigma subunit ($\sigma^{70}$) of E. coli RNA polymerase holoenzyme plays an essential role in regulating transcription initiation and maintaining cell growth. Several mutations in rpoD. the E. coli gene that encodes $\sigma^{70}$, including rpoD800 (rpoD285), lead to a temperature-sensitive growth phenotype (Ts).

Strains CAG510 (htpR+/rpoD800/supC[ts]) and CAG481 (htpR165/rpoD800/supC[ts]) were labeled for 10 minutes (min) with $^3$H-leucine and $^3$H-lysine during exponential growth at 30°. After addition of an excess ( > 200 µg/ml) of non-radioactive leucine and lysine, cultures were shifted to 42° and sampled periodically. The samples were analyzed for their content of rpoD800 encoded sigma on 2D gels as described by Grossman et al., Cell, 32, 151-159 (1983). The initial time point (t = O) was taken 2 min after addition of chase and was in the range of 2000-4000 dpm in sigma. At 42°, mutant sigma was rapidly degraded in the htpR+ strain (T 1/2 = 6 min) but was quite stable in the htpR165 conditional mutant strain (T 1/2 > 60 min). Thus, mutant sigma was stabilized at least 10 fold in htpR– mutant strains. The rate of degradation of mutant sigma was indistinguishable from that of wild type sigma in the htpR– mutant strain.

The rates of sigma degradation in isogenic htpR+ and htpR– mutant cells at 30° was compared because rpoD800 encoded sigma is degraded in wild type cells even at low temperature (See, Grossman et al., cited above). The mutant sigma polypeptide was degraded more slowly in the htpR– mutant strain (T 1/2 = 60 min) than in the htpR+ strain (T 1/2 = 20 min) at 30°.

B. Degradation of mutant β-galactosidase (X90).

Strains CAG603 (htpR+/F'lacZX90/supC[ts]) and CAG604 (htpR165/F'lacZX90/supC[ts]) growing exponentially at 30° in M9 glycerol media were induced with isopropyl-β-D-thiogalactopyranoside (IPTG), and shifted to 42° at 10 min after induction. At 20 min after transfer to 42°, cells were labeled with $^{35}$S-methionine for 1.5 min, chased with an excess ( >200µ g/ml) of nonradioactive methionine and sampled periodically. Samples were immunoprecipitated and analyzed for their content of β-galactosidase. The initial time point (t = 0) was taken 2 min after addition of chase and represents 5000 dpm in β-galactosidase.

The lacZX90 polypeptide was degraded considerably more slowly (T 1/2 >60 min) in CAG604, the htpR– mutant strain, than in the htpR+ strain (T 1/2 = 7 min) at 42°. Thus the lacZX90 polypeptide was stabilized at least 8.5 times in htpR– mutant strains. The rate of degradation of wild type lacZ gene product was indistinguishable from that of lacZX90 polypeptide in the htpR– mutant strain.

C. Degradation of rpoD800 gene product and lacZX90 gene product by htpR– mutant strains other than those with the htpR165 mutation.

It is important to note that the proteolytic defect manifested by htpR– mutant cells is not limited to those strains with the htpR165 mutation and the supCts gene. As disclosed by Grossman et al., J. Bacteriol., 161(3), 939-943 (1985), the same defect in the proteolysis of the rpoD800 (rpoD285) gene product is exhibited by htpR– (rpoH–) cells which have both a htpR107, htpR111, htpR112, or htpR113 mutation and the rpoD800 gene. Grossman et al. also observed the same defect in proteolysis of the lacZX90 gene product exhibited by htpR112 mutants carrying the rpoD800 gene. The htpR112 mutation is defined by a missense mutation causing a leucine-to-tryptophane change seven amino acids from the carboxyl terminus of the htpR (rpoH) gene product.

EXAMPLE 2 THE DEGRADATION DEFECT OF htpR– CONDITIONAL MUTANT STRAINS MAPS TO THE htpR LOCUS

The defect in proteolysis by htpR– mutant strains was mapped by a marker rescue experiment. The pFN92 plasmid, described by Neidhardt et al., J. Bacteriol., 153, 597-603 (1983) (Neidhardt I), carries a segment of bacterial DNA starting about 120 bp upstream of the N terminus of the htpR structural gene and ending within the htpR gene close to its 3' end [See, Neidhardt et al., Ann. Review. Genetics, 18, 295-329, (1984) ; and Landick et al., Cell, 38, 175-182 (1984)]. The pFN92 plasmid does not complement the htpR– defect. However, htpR+ recombinants which are temperature resistant and undergo a normal heat-shock response can be recovered from cells transformed by the pFN92 plasmid [See, Neidhardt I]. A htpR165 conditional mutant strain was transformed with pFN92 according to the method of Neidhardt I, and htpR+ recombinants were selected based on their temperature resistant phenotype. The ability of these strains (htpR165 conditional mutant, pFN92, nonrecombinant; htpR+, pFN92, recombinant) to undergo the heat-shock response and to degrade the lacZX90 polypeptide was compared. The non-recombinant strain retained the characteristics of the original htpR– mutant strain because it was defective in the heat-shock response and in the degradation of the lacZX90 polypeptide. In contrast, the recombinant strain simultaneously regained the ability to carry out the heat-shock response and the ability to degrade the lacZX90 polypeptide, both of which are characteristic of the htpR+ strain. It was also determined that htpR+ recombinant strains also regain the ability to degrade rpoD800 encoded mutant sigma. These experiments unambiguously map the defect of htpR– conditional mutant strains in the proteolysis of mutant prokaryotic gene products to a lesion in the htpR gene. Both a normal heat-shock response and degradation of the lacZX90 and rpoD800 encoded mutant sigma polypeptides require the htpR+ allele.

EXAMPLE 3 HEAT SHOCK PROTEIN SYNTHESIS AND PROTEIN DEGRADATION IN STRAINS RECOMBINANT (htpR+) AND NON-RECOMBINANT (htpR–) FOR THE htpR ALLELE

The experiments described in Example 3 were performed on strain CAG5107 (htpR– mutant/F'lacZX90 pFN92) and strain CAG5110 (hptR+/F'lacX90 pFN92). The hptR+ strain (CAG5110) was derived from the htpR– mutant strain (CAG5107) by a recombination event between pFN92 and the htpR– allele in the chromosome generating an htpR+ recombinant exhibiting ts+ growth at 42°. Transformation of CAG5110 with pFN92 was carried out prior to its selection by the procedure of Neidhardt I.

A. Restoration of the heat-shock response.

Strains CAG5107 and CAG5110 were pulse labeled at 30°, or 5 min after transfer to 42° with $^{35}$S methionine for 2 min and chased with non-radioactive methionine for 1 min. Samples were analyzed for the heat-shock response by electrophoresis on SDS-polyacrylamide gels. The slight heat-shock response in the nonrecombinant strain (CAG5107) was indistinguishable from that in the htpR– mutant strain, CAG456, and that observed from htpR– strains by other workers [See, Yamamori et al., PNAS, 79, 860-864 (1982), and Tilly et al., Cell, 34, 641-646 (1983)]. The heat-shock response in the recombinant strain (CAG5110) was indistinguishable from that in the htpR+ strain SC122.

B. Degradation of mutant β-galactosidase (lacZX90 polypeptide).

Strains CAG5107 and CAG5110, growing exponentially at 30° in M9 glycerol medium, were induced with IPTG and shifted to 42° 10 min after induction. At 20 min after transfer to 42°, cells were labeled with $^{35}$S methionine for 1.5 min, chased with an excess of non-radioactive methionine and sampled periodically. Samples were analyzed on one dimensional gels for their content of β-galactosidase as a fraction of $\beta + \beta'$ subunits. The initial time point (t=0) was taken 2 min after addition of chase and represents 5000 dpm in β-galactosidase. The htpR– mutant strain degraded the lacZX90 gene product at a much slower rate (T 1/2 d > 60 min) than the htpR+ strain (T 1/2 = 7 minutes).

C. Degradation of mutant sigma (encoded by the rpoD800 sigma allele) in htpR- strains with and without the supF suppressor (SulII).

The supCts suppressor present in htpR165 conditional mutant strains, such as K165, CAG456, CAG481 and CAG482. is an inefficient suppressor of the htpR165 mutation. Thus, the defect in proteolysis observed in such htpR- mutant cells at 42° could be due to a lower amount of the htpR product synthesized at 30° rather than the lack of either the heat-shock response or htpR synthesis at 42°. In the former case, the defect in proteolysis exhibited by htpR- mutant cells should occur at 30° as well as at 42°. The rates of rpoD800-encoded sigma subunit degradation in isogeneic htpR+ and htpR- mutant cells was compared at 30° because rpoD800-encoded sigma subunit is degraded in wild-type cells even at low temperature. The ability of the efficient supF (SulII) suppressor to restore the ability of htpR165 conditional mutant cells to carry out proteolysis at 42° was also examined.

Degradation of mutant sigma was measured in three strains: strain CAG510 (htpR+, rpoD800), strain CAG483 (htpR165 mutant, 80pSulII, rpoD800), and strain CAG482 (htpR165 mutant, 80, rpoD800). CAG482 is a control to demonstrate that genes in 80 do not affect the proteolysis phenotype of htpR- mutant cells. Cells growing exponentially at 30° were labeled with 35S-methionine for 10 min. After addition of an excess (1 mg/ml) of non-radioactive methionine, cultures were shifted to 42° or left at 30° and sampled periodically. The samples were analyzed for their content of rpoD800-encoded sigma. The initial time point (t=0) was taken 2 min after addition of chase and is in the range of 5,000-10,000 dpm in sigma.

The rpoD800-encoded mutant sigma protein was degraded more slowly in the htpR- mutant strains than in the htpR+ strain at 30°. Rapid degradation at 30° was restored when the more efficient supF (SulII) suppressor was present. It was also determined that the supF (SulII) suppressor restored the ability of htpR165 conditional mutant cells to carry out proteolysis at 42°, along with restoring the ability to carry out synthesis of heat-shock proteins. Thus, increasing the level of suppression simultaneously restored both a normal heat-shock response and proteolysis.

These experiments show that inefficient suppression of the htpR amber mutation (i.e., htpR165) is sufficient to cause the proteolysis defect both at low temperatures under steady state growth conditions as well as after shift to high temperature.

EXAMPLE 4 STABILIZATION OF EXPRESSION OF TWO DIFFERENT FORMS OF HUMAN c-myc IN A htpR- STRAIN

A. Bacterial strains and plasmids.

The host used for construction of the expression vector was E. coli MM294 (λ cl+). E. coli AR68 was utilized for protein expression studies. The plasmid vector, pOTS, is a derivative of the expression vector pAS1, and is described by Devare et al., Cell, 36, 43-49 (1984).

All standard molecular cloning techniques (e.g., restriction digests, ligations, transformation, etc.) were carried out according to the method of Maniatis et al., "Molecular Cloning, A Laboratory Manual", Cold Spring Harbor Laboratory (1982), unless otherwise noted.

B. Construction of pOTS-myc, a c-myc expression plasmid.

The protein-coding sequence of the c-myc oncogene was obtained from a cDNA clone (pMYClaC), prepared according to the procedure of Watt et al., PNAS, 80, 6307-6311 (1983), which had been isolated from a cDNA library prepared from the human erythroleukemic cell line K562. The 5' end of the c-myc coding sequence was isolated on a 470-base pair ThaI restriction fragment by agarose gel electrophoresis, and then ligated into the pOTS vector which had been digested with BamHI and blunt-ended using the Klenow fragment of DNA polymerase I. This plasmid construction was digested partially with PvuII, followed by digestion with XbaI and end-filling with Klenow PolI. The plasmid construction, which had been linearized by digestion of only the PvuII site within the ThaI fragment, was isolated by agarose gel electrophoresis. The 3' end of the c-myc coding sequence was isolated on an 1,100-base pair PvuII/BclI fragment and was ligated into the partially digested construction described above. The pOTS-myc expression plasmid thus generated contains the entire c-myc coding region fused in-frame with the translation initiation codon provided on the pOTS vector. The resulting construction contains four additional codons on the 5' end of the c-myc coding sequence.

C. Analysis of induced bacterial proteins.

The rate of synthesis of proteins in E. coli strain AR68, a htpR- mutant, transformed with pOTS-myc or pOTS. according to the method of Maniatis et al., cited above, both before and after induction at 42° was studied by pulse-labeling according to the method of Rosenberg et al., Methods Enzymol., 101, 123-138 (1983). Aliquots (200 1) of cells were pulse-labeled for 3 min with 10 Ci of [35S] methionine (1,221 Ci/mmol; New England Nuclear) at various times after thermal induction. After pulse-labeling, 30 μ l of 10% sodium dodecyl sulfate (SDS)-35% 2-mercaptoethanol was added, and the samples were heated at 95° for 1 min, followed by quick freezing in a dry ice-ethanol bath, and precipitated with trichloroacetic acid (final concentration, 10%). The precipitates were washed twice with cold ethanol, suspended in sample buffer, heated at 95° for 5 min, and subjected to electrophoresis on a 10% polyacrylamide gel. The gels were finally dried and fluorographed. The samples were normalized for neither cell number nor number of counts loaded on the gel.

The results of analysis of the gels indicated that the synthesis of a new 64,000-dalton protein (i.e. c-myc) was induced in cells transformed with pOTS-myc, but was not detected in cells transformed with the parent vector pOTS. This 64,000-dalton protein was expressed at a high rate since a significant portion of the [35S] methionine incorporated into proteins within the 3-min pulse period appears in the c-myc gene product. To examine whether this protein could accumulate to high levels in the bacteria, cells were induced for up to 120 min, and aliquots taken at different times were examined for c-myc production by SDS-gel electrophoresis. Coomassie blue staining of the gel showed that the induced product had accumulated to over 10% of the total E. coli protein during the 2 hour (h) time period.

The 64,000 dalton protein produced by the AR68 transformants was verified as c-myc protein by amino acid analysis and sequence determination in accordance with the procedures of Spackman et al., Anal. Chem., 30. 1190-1206 (1958) and Hewick et al., J. Biol. Chem., 256, 7990-7997 (1981).

D. Expression of c-myc by htpR+ strains.

Three htpR+ strains (i.e., N5151, AR66 and AR58) were transformed with the pOTS-myc vector as described above. Although all strains were transformed successfully with the pOTS-myc vector, as indicated by SDS-gel electrophoresis of pulse-labeled E. coli extracts, the htpR+ transformants were unable to stably express the c-myc product since such product could not be detected by Coomassie blue staining of the gel. Since c-myc was expressed stably in AR68 (htpR– mutant) but not in its isogen, AR66 (htpR+), the htpR– mutation present in AR68 was essential for the accumulation, and thus stabilization, of the c-myc gene product in E. coli.

Therefore, the htpR– mutation was necessary to enable stable expression of the c-myc coding sequence in E. coli hosts transformed therewith.

EXAMPLE 5 STABILIZATION OF EXPRESSION OF THE YEAST GAL4 GENE PRODUCTS (AUTHENTIC PROTEIN OR TRUNCATED VARIANTS) IN A htpR– CONDITIONAL MUTANT STRAIN

A. Construction of a bacterial vector containing the coding sequence for authentic yeast GAL4 protein.

The yeast GAL4 coding region, lacking the first 32 bp at its 5' end, was obtained as an SphI-HindIII restriction endonuclease fragment from plasmid pSJ4 [See, Johnston et al., Proc. Natl. Acad. Sci. USA, 79, 6971-6975 (1982)]. Prior to SphI cleavage, the HindIII site was first blunt-ended by treatment with DNA polymerase I (Klenow). This fragment was then isolated by polyacrylamide gel electrophoresis, and inserted between the SphI and NruI sites of the pAS1 expression vector to yield pCD106. pAS1 is described in Rosenberg et al., Methods Enzymol., 101, 123-138 (1983). Plasmid pCD106 was digested with BamHI, followed by treatment with Mung Bean exonuclease to create a blunt-end cloning site immediately adjacent to the initiation codon provided on the pAS1 vector [See, Rosenberg et al., cited above]. The plasmid was then recut with SphI, and a synthetic DNA linker that reconstructed the entire amino-terminal coding sequence of GAL4 extending from the second codon to the SphI site was inserted. The nucleotide sequence of the linker was made to differ somewhat from the DNA sequence of the GAL4 gene, however the amino acid sequence of the protein was not altered. Seven nucleotide changes were made to improve the codon usage in E. coli [see, Grosjean et al., Gene, 18, 199-209 (1982)], and to generate additional restriction sites in the amino-terminal end of the gene (SacI, PvuI). The final construction, pCD107, consists of the reconstructed full-length GAL4 coding sequence positioned in-frame with the translation initiation codon provided on pAS1.

B. Construction of bacterial vectors containing the coding sequence for yeast GAL4 deletion mutants.

The pAS1 expression vector readily allows the construction and expression of truncated derivatives of any gene. Since the vector supplies the ribosome binding site as well as the ATG initiation codon, any sequence placed in-frame with this ATG has the potential to be expressed. This versatility was utilized to create several truncated derivatives of the yeast GAL4 coding sequence.

Plasmid pCD93 carries an amino-terminal deletion removing the first 74 amino acids of GAL4. It was obtained from pCD106, prepared as described above, by BamHI and XhoI digestion, followed by treatment with Mung Bean exonuclease and religation. This procedure precisely fused the 75th codon (at the XhoI site) of GAL4 to the ATG initiation codon (at the BamHI site) provided on pAS1.

Plasmids pCD111 and pCD144 were constructed from pCD107, prepared as described above, and carry carboxy-terminal deletions extending, respectively, from the 414th codon (pCD111) or the 334th codon (pCD144) of GAL4 to the end of the protein. pCD111 was obtained from pCD107 by initial digestion with SalI. The resulting four-base recessed 3' ends were then filled-in with DNA polymerase I (Klenow fragment), and the plasmid was recircularized by ligation. This procedure shifted the GAL4 translation reading frame at the filled-in SalI site to the +1 reading frame which stops translation prematurely only a few triplets after the 414th codon of GAL4. Plasmid pCD144 was generated in a similar way from pCD107 by initial digestion with NruI and SalI, followed by treatment with DNA polymerase I to fill-in the SalI recessed 3' ends and religation. This procedure fused the 333d codon (at the NruI site) of GAL4 to the prematurely terminated +1 reading frame (at the filled-in SalI site), resulting in a GAL4 truncated derivative lacking amino acids 334 to 881.

Plasmid pCD98 was derived from pCD93, prepared as described above, and carries an amino- as well as a carboxy-terminal deletion of the GAL4 coding sequence. The protein expressed from this plasmid consists of amino acids 75 to 413 of GAL4. pCD98 was obtained from pCD93 by the same manipulation described above for the construction of pCD111.

Finally, plasmids pCD151 and pCD150 each carry an internal deletion in the GAL4 coding sequence. These plasmids were obtained from pCD107, prepared as described above, by restriction with XhoI and either NruI (pCD151) or HpaI (pCD150), followed by treatment with DNA polymerase I to fill-in the XhoI recessed 3' ends and religation. This manipulation created an in-frame deletion of 259 codons (75 to 334, pCD151) or 147 codons (75 to 222, pCD150) from the GAL4 coding sequence.

C. Transformation of htpR- mutant and htpR+ strains with bacterial vectors containing the coding sequence for authentic or truncated yeast GAL4 protein.

The pAS1-GAL4 constructs, prepared as described in parts A and B above, were introduced into AR65, AR66, AR67 (htpR- mutant, isogenic to htpR+ strain AR65), and AR68 (htpR- mutant, isogenic to htpR+ AR66) by the method of Maniatis et al., "Molecular Cloning", Cold Spring Harbor Laboratory. Transformants were selected based on their expression of ampicillin resistance.

D. Analysis of induced GAL4 production.

The $P_L$ promoter of the expression vectors contained by transformants, prepared as described above, was induced by temperature shift. The proteins produced were labeled with $^{35}$S-methionine according to the procedure of Rosenberg et al., Meth. Enzymol., 101, 123-138 (1983). Thirty minutes after heat induction, the transformants were labeled for two minutes with 100 microcuries of $^{35}$S-methionine, and then an equal volume of 2 mM cold methionine and 500 µg/ml chloramphenicol was added. Aliquots were removed at various times to assess protein stability after SDS-polyacrylamide gel electrophoresis (pulse chase experiments) according to the method of Rosenberg et al., cited above.

Analysis of the gels revealed that all transformants (both htpR- mutants and htpR+) produced either authentic or truncated GAL4 product, but that expression of both the authentic and truncated product was significantly stabilized in htpR- mutant strains since only the htpR- conditional mutant strains showed accumulation of the gene product upon heat induction.

EXAMPLE 6 STABILIZATION OF EXPRESSION OF MUTANT cII CODING SEQUENCES IN A htpR-CONDITIONAL MUTANT STRAIN

cII protein of bacteriophage λ is a positive regulatory protein of transcription. The cII gene, and more than 30 of the point mutations of the cII protein which cover the different regions of the entire cII structural gene, were cloned in pKC30, and transformed into lysogens for gene expression by heat induction.

A. Production of cII mutant coding sequences.

All the cII mutant coding sequences used were generated and sequenced according to the method of Wulff et al., Proc. Natl. Acad. Sci. USA, 81, 555-559 (1984). All the cII mutants examined had one point mutation (i.e., single amino acid sequence change) caused by a base pair substitution in the structural gene of cII protein. The wild type cII protein and mutant cII proteins were purified according to the method of Ho et al., J. Biol. Chem., 257(15), 9128-9134 (1982).

B. Construction of expression vectors containing the cII coding sequence or a mutated cII coding sequence.

The cII coding sequence was cloned into an expression vector, pKC30, by the method of Shimatake et al., Nature. 292(5819), 128-132 (1981). The 30 mutated cII coding sequences, prepared as described in part A, were cloned into pKC30 basically by the method of Shimatake et al., cited above, except that the 0 protein coding sequence was eliminated from the DNA cloning fragment to facilitate the purification procedure.

C. Transformation of htpR+ and htpR- cell lines.

The expression vectors, prepared as described in part B, were transformed into AR67 (htpR-mutant) and UC5822 (htpR+) according to the method of Maniatis et al., "Molecular Cloning, A Laboratory Manual", Cold Spring Harbor Laboratory (1982). Several of the expression vectors were also transformed into other htpR+ cell lines such as AR65, AR58, AR13 and N5151, according to the method of Maniatis et al., cited above.

D. Analysis of induced proteins.

The stability of cII protein or mutated cII protein expressed by the transformants, prepared as des cribed in part C, after induction at 42°, was studied by the S$^{35}$-L-methionine pulse-chase procedure of Rosenberg et al., Methods Enzymol., 101, 123-138 (1983). Wild type cII protein was expressed at the same level of instability in AR67 (htpR- mutant), AR65 (htpR+) and UC5822 (htpR+). Mutated cII protein was not nearly as stably expressed in UC5822 (htpR+) as was the wild type cII protein. The half-life of most of the cII mutant proteins in UC5822 was 3-5 min versus 20 min for the wild type cII protein. Some of the mutant cII proteins had a half-life of < 1 min in UC5822. None of the other htpR+ cell lines employed, i.e., AR65, AR58, AR13 and N5151, gave significantly different results in the half-life or net protein accumulation of the mutant cII proteins. The stability of all the mutant cII proteins was significantly enhanced in AR67 (htpR– mutant, isogenic to htpR+ strain AR65), i.e. increased to the same level of stability as the wild type cII protein. For example, the half-life of a very unstable mutant cII protein, cII3085, is extended from 0.5 min in htpR+ cell lines including AR65 to ~ 20 min in its isogen AR67 (htpR– mutant).

While the above descriptions and Examples fully describe the invention and the preferred embodiments thereof, it is understood that the invention is not limited to the particular disclosed embodiments. Thus, the invention includes all embodiments coming within the scope of the following claims.

## Claims

1. A method of stably expressing a foreign polypeptide coding sequence not stably expressed in E. coli htpR+ strains due to degradation by proteases under the control of the htpR$^R$ gene which comprises (a) transforming a host E. coli htpR mutant cell with a DNA molecule containing the polypeptide coding sequence operatively linked to an expression control sequence, and (b) culturing the transformed host in an appropriate medium under conditions such that the coding sequence is expressed.

2. The method of Claim 1 wherein the host htpR– cell is a htpR165 strain also carrying a supC$^{ts}$ gene; or a htpr107 strain, a htpR111 strain, a htpR112 strain or a htpR113 strain also carrying a rpoD800 gene.

3. The method of Claim 2 wherein the host htpR– mutant cell is K165, CAG456, CAG481, CAG482, CAG483, CAG604, AR67 or AR68.

4. The method of Claim 1 wherein the expression control sequence is the P$_L$ promoter.

5. The method of Claim 1 wherein the DNA molecule is derived from a P$_L$ vector such as pAS1 or pKC30.

6. A host E. coli htpR– mutant cell transformed with a DNA molecule containing a foreign polypeptide coding sequence operatively linked to an expression control sequence.

7. The host of Claim 6 which is htpR165 strain also carrying a supC$^{ts}$ gene.

8. The host of Claim 7 wherein the htpR165 strain is K165, CAG456, CAG481, CAG482, CAG483, CAG604, AR67 or AR68.

9. The host of Claim 6 which is htpR107 strain, a htpR111 strain, a htpR112 strain or a htpR113 strain also carrying a rpoD800 gene.

10. The host of Claim 6 wherein the DNA molecule is derived from a P$_L$ vector such as pAS1 or pKC30.

11. The host of Claim 6 wherein the expression control sequence is the T$_L$ promoter.

12. A method of stably expressing a foreign polypeptide coding sequence not stably expressed in E. coli htpR+ strains due to degradation by proteases under the control of the htpR+ gene which comprises (a) transforming a host E. coli htpR+ cell with a DNA molecule containing the polypeptide coding sequence operatively linked to an expression control sequence, (b) changing the phenotype of the transformed htpR+ cell to a htpR– mutant, and (c) culturing the transformed htpR– mutant in an appropriate medium under conditions such that the coding sequence is expressed.

13. The method of Claim 12 wherein the phenotype of the htpR+ cell is changed to a htpR165 strain, a htpR111 strain, a htpr112 strain or a htpR113 strain also carrying a rpoD800 gene.

14. The method of Claim 13 wherein the htpR165 strain is K165, CAG456, CAG481, CAG482, CAG483, CAG604, AR67 or AR68.

15. The method of Claim 12 wherein the expression control sequence is the P$_L$ promoter.

16. The method of Claim 12 wherein the DNA molecule is derived from a P$_L$ vector such as pAS1 or pKC30.

17. A method of producing a host E. coli htpR mutant cell transformed with a DNA molecule containing a foreign polypeptide coding sequence operatively lin ked to an expression control sequence which comprises transforming a host E. coli htpR– mutant cell with the DNA molecule.

18. The method of Claim 17 wherein the host is a htpR165 strain also carrying a supC$^{ts}$ gene.

19. The method of Claim 18 wherein the host is htpR165 strain K165, CAG456, CAG481, CAG482, CAG483, CAG604, AR67 or AR68.

20. The method of Claim 17 wherein the host is a htpR107 strain, a htpR111 strain, a htpR112 strain or a htpR113 strain also carrying a rpoD800 gene.

21. The method of Claim 17 wherein the expression control sequence is the P$_L$ promoter.

22. The method of Claim 17 wherein the expression control sequence is derived from a P$_L$ vector such as pAS1 or pKC30.

CLAIMS FOR THE CONTRACTING STATE AT

1. A method of stably expressing a foreign polypeptide coding sequence not stably expressed in E. coli htpR+ strains due to degradation by proteases under the control of the htpR$^R$ gene which comprises (a) transforming a host E. coli htpR mutant cell with a DNA molecule containing the polypeptide coding sequence operatively linked to an expression control sequence, and (b) culturing the transformed host in an appropriate medium under conditions such that the coding sequence is expressed.

2. The method of Claim 1 wherein the host htpR- cell is a htpR165 strain also carryinga supC$^{ts}$ gene; or a htpr107 strain, a htpR111 strain, a htpR112 strain or a htpR113 strain also carrying a rpoD800 gene.

3. The method of Claim 2 wherein the host htpR- mutant cell is K165, CAG456, CAG481, CAG482. CAG483, CAG604, AR67 or AR68.

4. The method of Claim 1 wherein the expression control sequence is the P$_L$ promoter.

5. The method of Claim 1 wherein the DNA molecule is derived from a P$_L$ vector such as pAS1 or pKC30.

6. A method of stably expressing a foreign polypeptide coding sequence not stably expressed in E. coli htpR+ strains due to degradation by proteases under the control of the htpR+ gene which comprises (a) transforming a host E. coli htpR+ cell with a DNA molecule containing the polypeptide coding sequence operatively linked to an expression control sequence, (b) changing the phenotype of the transformed htpR+ cell to a htpR- mutant, and (c) culturing the transformed htpR- mutant in an appropriate medium under conditions such that the coding sequence is expressed.

7. The method of Claim 6 wherein the phenotype of the htpR+ cell is changed to a htpR165 strain, a htpR111 strain, a htpr112 strain or a htpR113 strain also carrying a rpoD800 gene.

8. The method of Claim 7 wherein the htpR165 strain is K165, CAG456, CAG481, CAG482, CAG483, CAG604, AR67 or AR68.

9. The method of Claim 6 wherein the expression control sequence is the P$_L$ promoter.

10. The method of Claim 6 wherein the DNA molecule is derived from a P$_L$ vector such as pAS1 or pKC30.

11. A method of producing a host E. coli htpR mutant cell transformed with a DNA molecule containing a foreign polypeptide coding sequence operatively linked to an expression control sequence which comprises transforming a host E. coli htpR- mutant cell with the DNA molecule.

12. The method of Claim 11 wherein the host is a htpR165 strain also carrying a supC$^{ts}$ gene.

13. The method of Claim 12 wherein the host is htpR165 strain K165, CAG456, CAG481, CAG482, CAG483, CAG604, AR67 or AR68.

14. The method of Claim 11 wherein the host is a htpR107 strain, a htpR111 strain, a htpR112 strain or a htpR113 strain also carrying a rpoD800 gene.

15. The method of Claim 11 wherein the expression control sequence is the P$_L$ promoter.

16. The method of Claim 11 wherein the expression control sequence is derived from a P$_L$ vector such as pAS1 or pKC30.